# EUROPEAN PATENT SPECIFICATION

(11) **EP 2 368 499 B1**
(45) Date of publication and mention of the grant of the patent: **02.08.2017**
(21) Application number: 09834951.7
(22) Date of filing: 24.12.2009
(51) Int. Cl.: A61B 17/00, A61M 5/14

(54) **TREATMENT TOOL**
BEHANDLUNGSGERÄT
APPAREIL DE TRAITEMENT

(30) Priority: 24.12.2008 JP 2008327379
(43) Date of publication of application: 28.09.2011
(73) Proprietor: Olympus Corporation, Tokyo 192-8507 (JP)
(72) Inventor: SATO, Kazuya, Hachioji-shi, Tokyo 192-8507 (JP); ONODERA, Yuta, Hachioji-shi, Tokyo 192-8507 (JP); KIMURA, Koh, Hachioji-shi, Tokyo 192-8507 (JP)
(74) Representative: Schicker, Silvia
(86) International application number: PCT/JP2009/071453
(87) International publication number: WO 2010/074153

(56) References cited:
- WO-A1-2007/046444
- WO-A1-2007/046444
- JP-A- 7 289 636
- JP-A- 2001 058 006
- JP-A- 2007 229 102
- JP-A- 2008 272 365
- JP-T- 8 509 628

## Description

### Technical Field

The present invention relates to a treatment tool which is inserted through an endoscope or the like for performing various kinds of treatment on a tissue in a body cavity.

Priority is claimed on Japanese Patent Application No. 2008-327379 filed on December 24, 2008, the contents of which are incorporated herein by reference.

### Background Art

Conventionally, treatment instrument endoscopically inserted into a body cavity in order to perform various kinds of treatment on a tissue in the body cavity is known. Generally, these treatment tools have an elongated insertion portion having flexibility, and a treatment part which performs treatment is provided at the tip of an insertion portion inserted into a body. An operation part for operating the treatment part is provided at the proximal side of the insertion portion which is pulled out from the proximal side of an endoscope or the like through which the treatment tool is inserted. The treatment part and the operation part are connected together by a connecting member, such as a wire or a rod.

Although the shape or construction of the treatment part may vary greatly depending on the procedures to be performed by the treatment tool, a treatment tool including a treatment part with a hollow needle tube with a sharp tip is known as an example (for example, refer to PTL 1). Such a treatment tool is used, for example, when a medicinal solution is injected into a tissue, or a saline solution or the like is injected into a tissue to make the tissue swell.

In the treatment tool described in PTL 1, a tube which supplies fluids, such as a medicinal solution, is attached to a proximal end of the needle tube, and is inserted into an external tubular sheath which constitutes an insertion portion. The needle tube is integrally fixed to the tube via a coupling member made of metal or the like. Since a comparatively large force acts on the needle tube when the needle tube is inserted into a tissue, it is necessary to reliably fix the needle tube and the coupling member. In practice, both the needle tube and the coupling member are often fixed, for example, by caulking the coupling member as this is simple and easy. In addition, the coupling member may be fixed not only by caulking but also by brazing.

### Patent Document

[Patent Document 1] Japanese Unexamined Patent Application, First Publication No. 2008-173313

Furthermore, WO 2007/046444 A1 dislcoses an injection needle for an endoscope, in which an insert part is formed with a double tube having an inner tube and an outer tube, the material for the outer tube being harder than that for the inner tube.

### Summary of Invention

### Technical Problem

However, the coupling member is substantially cylindrical before working, but the radial section thereof becomes substantially elliptical due to a fixing force during fixing. As a result, since the radial dimension of the coupling member in a direction orthogonal to the pressing direction of the connecting member is increased, it is necessary to insert the connecting member through a sheath capable of receiving the increased diameter.

If the treatment tool of Patent Document 1 is constructed in this way, there is a problem in that a gap may be generated between the coupling member and the inner cavity of the sheath, and this gap may cause wobbling of the needle tube in the axial direction during treatment.

Although both the connecting member and the sheath can be fixed using an adhesive or the like, there is a problem in that the fixing task becomes complicated, and the adhesion strength is also insufficient.

The invention has been made in consideration of the above circumstances, and the object thereof is to provide a treatment tool in which wobbling does not easily occur in a direction where the treatment part or the connecting member separates from the axis.

In addition, a fixing method of a coupling member and a needle tube is not limited to caulking and brazing, but other fixing methods may be employed. In the following description, a case where fixing is performed by caulking and brazing is described as an example.

### Solution to Problem

The above object is solved with the treatment tool according to claim 1 and the further advantageous embodiments according to the dependent claims.

A further aspect of the invention is a treatment tool endoscopically inserted into a body cavity, including a treatment part performs a treatment within the body cavity; a connecting member connected to a proximal end of the treatment part; a sheath through which the treatment part and the connecting member are inserted so as to be able to extend or retract in an axis direction; an operation part having the connecting member connected thereto; and a coupling member fixed to at least one of the connecting member and the treatment part. The coupling member has a large diameter portion having an external diameter substantially equal to the internal diameter of the sheath, and a fixing portion having an external diameter smaller than the large diameter portion, and is formed in a tubular shape, the fixing portion is fixed to at least one of the connecting member and the treatment part, and the radial maximum dimension of the fixing portion is less than or equal to the external diameter of the large diameter portion in a fixed state.

According to the treatment tool of the invention, the coupling member is integrally attached to the treatment part or the connecting member by fixing the fixing portion by caulking, brazing, or the like. However, since the radial dimension of the coupling member becomes the greatest at the large diameter portion even after fixing, a large gap does not exist between the large diameter portion and the sheath, and the treatment part or the connecting member is suitably prevented from wobbling, such as separating from the axis, during operation.

The treatment part and the connecting member may be integrally connected by the coupling member. In this case, the treatment part and the connecting member can be reliably connected by caulking or brazing while suppressing wobbling within the sheath.

The sheath may have an abutting portion which is formed so that the treatment part can pass therethrough and the coupling member cannot pass therethrough. In this case, as the connecting member is extended with respect to the sheath until the coupling member abuts on the abutting portion, the coupling member can function as a stopper, and the projecting amount control or positioning of the treatment part can be suitably performed.

The abutting portion may be formed by reducing the internal diameter of the tip of the sheath. In this case, the abutting portion can be easily formed in the sheath.

The large diameter portion may have a first large diameter portion at the distal side, and a second large diameter portion located closer to the proximal side than the first large diameter portion, and the fixing portion may be provided between the first large diameter portion and the second large diameter portion. In this case, wobbling of the treatment part or the connecting member is further reduced, and the behavior thereof can be further stabilized.

The treatment part may be a hollow needle tube, and the connecting member may be formed in the shape of a tube capable of supplying a fluid to the needle tube. In this case, a fluid can be supplied in a state where the behavior of the needle tube is stable, and treatments, such as injection of a medicinal solution, tissue swelling, or the like, can be performed.

### Advantageous Effects of Invention

According to the treatment tool of the invention, it is possible to provide a construction in which wobbling does not easily occur in a direction where the treatment part or the connecting member separates from the axis.

### Brief Description of Drawings

FIG 1 is a view showing a treatment tool of a first embodiment of the invention.
FIG. 2 is an enlarged sectional view in the vicinity of the tip of the treatment tool..
FIG 3 is a view showing a coupling member of the treatment tool.
FIG 4 is a view showing the process of fixing the coupling member and needle tube of the treatment tool integrally.
FIG 5 is a view showing the coupling member and needle tube after fixing.
FIG 6 is a sectional view showing an operation part of the treatment tool.
FIG 7 is a sectional view in a line A-A of FIG 6.
FIG 8 is a sectional view showing the operation part when a treatment part is housed in a sheath.
FIG 9 is an enlarged sectional view in the vicinity of the tip of a treatment tool of a second embodiment of the invention.
FIG. 10 is an enlarged sectional view in the vicinity of the tip of a treatment tool of a modification of the invention.
FIG 11 is an enlarged sectional view around an operation part of the treatment tool of the modification of the invention.

### Description of Embodiments

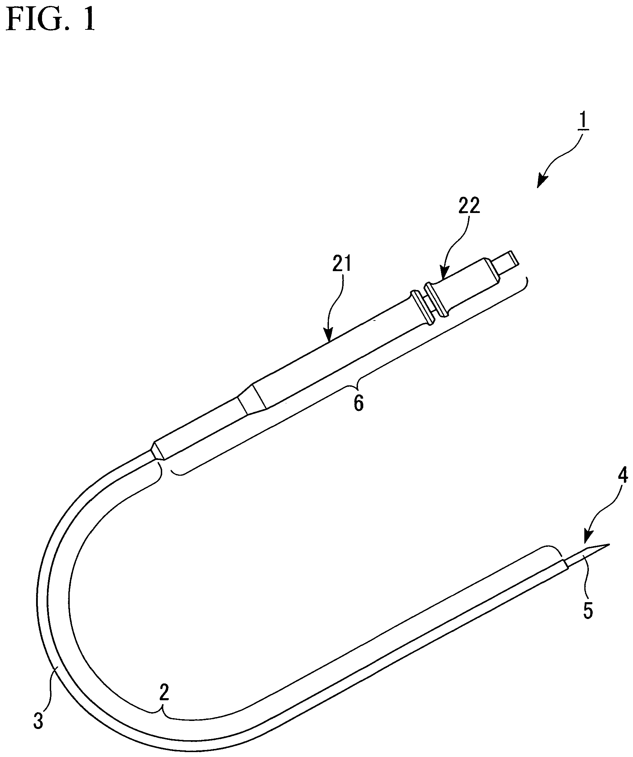

A treatment tool of a first embodiment of the invention will be described with reference to FIGS. 1 to 8.

FIG 1 is a view showing the treatment tool 1 of the present embodiment. The treatment tool 1 is constructed such that a hollow needle tube 5, which becomes a treatment part 4, is provided at the tip of an elongated sheath 3, which becomes an insertion portion 2 inserted into a body cavity, so as to freely project or recede from the tip. An operation part 6 for performing the projecting/receding operation of the needle tube 5 is attached to a proximal end of the sheath 3.

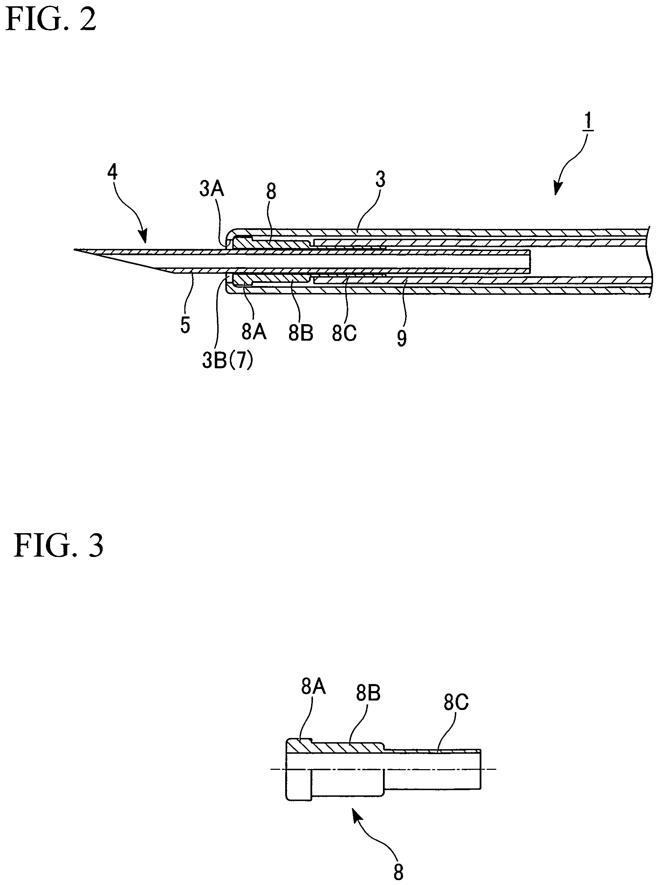

FIG 2 is an enlarged sectional view showing the distal side of the treatment tool 1. The sheath 3 is formed using resin or the like so as to have flexibility, and an opening 3A at the distal side of the sheath has a reduced opening diameter to such a degree that the needle tube 5 can be inserted therethrough, and a coupling member which will be described later cannot be inserted therethrough. An end face 3B in the opening 3A functions as an abutting portion 7 which regulates the projecting length of the needle tube 5 from the sheath 3 to a predetermined length.

The proximal side of the needle tube 5 is connected to a tube (connecting member) 9 made of resin or the like via a tubular coupling member (sliding member) 8 made of metal. A proximal end of the tube 9 is connected to the operation part 6 and transmits the projecting/receding operation of the needle tube 5 in the operation part 6 to the needle tube 5, and also functions as a supply means which supplies fluids, such as a medicinal solution and air, to the needle tube 5. The needle tube 5, the coupling member 8, and the tube 9 are inserted through the sheath 3 so as to be able to extend and retract in an axis direction.

FIG. 3 is a view showing the coupling member 8. The coupling member 8 is a substantially cylindrical member formed from a plastically deformable material, such as metal. The coupling member 8 has a large diameter portion 8A at the distal side with the largest external diameter, a fixing portion 8B provided at the proximal side of the large diameter portion 8A, and a connecting portion 8C provided at the proximal side of the fixing portion 8B. The external diameter of the large diameter portion 8A is set to be equal to or slightly smaller than the internal diameter of the sheath 3, i.e., substantially equal to the internal diameter of the sheath 3. Accordingly, the coupling member 8 having the large diameter portion 8A, as shown in FIG 2, is not able to pass through the opening 3A of the tip of the sheath 3, and abuts the abutting portion 7.

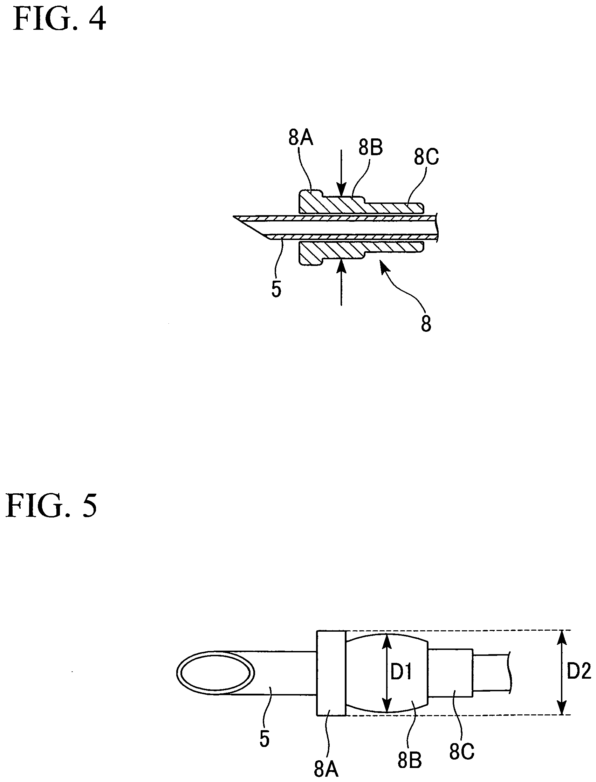

As shown in FIG. 4, the needle tube 5 is inserted through the coupling member 8, and the fixing portion 8B is pressed from the direction of the arrow, and is plastically deformed. That is, the needle tube 5 and the coupling member 8 are integrally fixed as the fixing portion 8B is caulked.

In addition, the pressing direction shown by the arrow in FIG. 4 is an example, and the direction is not particularly limited.

In addition, the needle tube 5 and the coupling member 8 may be integrally fixed by not only caulking but also brazing. Although not shown, in the case of brazing, a through hole penetrating in the radial direction to the needle tube is formed in a portion of the fixing portion of the coupling member. As brazing filler metal which flows in from this through hole fills and solidifies a gap between the needle tube and the coupling member, the needle tube and the coupling member are integrally fixed.

Additionally, in the case of brazing, stainless steel and steel suitable for brazing among metals may be used for the material of the coupling member.

Although the fixing portion 8B before caulking is substantially cylindrical, the cross-section of the fixing portion orthogonal to the axis direction becomes substantially elliptical due to the caulking. As a result, as shown in FIG 5, the radial dimension of the fixing portion 8B increases partially. The external diameter before the caulking of the fixing portion 8B is set to be smaller than the external diameter of the large diameter portion 8A, and the pressing force is adjusted to perform caulking so that the maximum external diameter D1 of the fixing portion 8B at this time becomes less than or equal to the external diameter D2 of the large diameter portion 8A.

The needle tube 5 and the coupling member 8 which have been integrally fixed, as shown in FIG 2, are integrally fixed with an adhesive or the like after the connecting portion 8C is inserted into the distal side of the tube 9. In this way, the needle tube 5, the coupling member 8, and the tube 9 are integrated and connected together so as to be able to extend and retract with respect to the sheath 3.

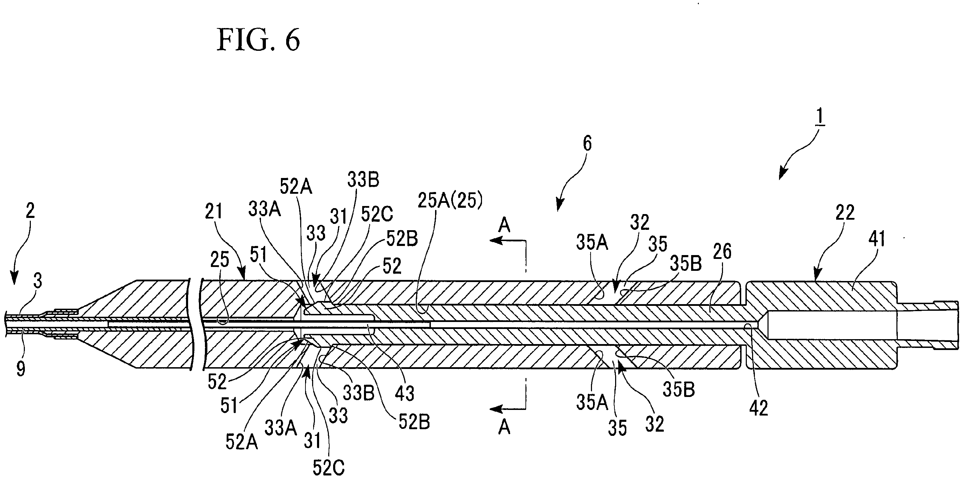

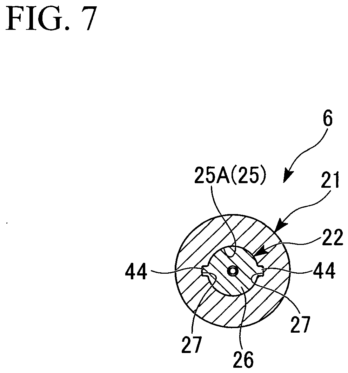

As shown in FIGS. 1 and 6, the operation part 6 has an operation part body 21 to which the proximal end of the sheath 3 is connected and fixed, and an operation tube 22 which is extendably and retractably inserted into the operation part body 21. The operation part body 21 is formed with a hole 25 which penetrates in the longitudinal direction (axial direction), and the tube 9 is inserted into the hole 25. The diameter of the hole 25 is made larger at the proximal side than at the distal side, and a tube portion 26 of the operation tube 22 is inserted into the diameter-enlarged hole 25A. As shown in FIG 7, the diameter-enlarged hole 25A is formed with two key grooves 27 which extend in the longitudinal direction. The number and arrangement of the key grooves 27 are not limited to those illustrated.

Moreover, a first diameter-enlarged portion 31 and a second diameter-enlarged portion 32 of which the internal diameter is enlarged are respectively formed at the distal side and proximal side of the diameter-enlarged hole 25A so as to avoid the key grooves 27, respectively. A pair of the first diameter-enlarged portions 31 at the distal side is symmetrically provided across the hole 25A, and includes holes 33 which are bored toward the diameter-enlarged hole 25A from the outer periphery of the operation part body 21.

Each hole 33 is bored obliquely with respect to the axis of the operation part body 21 so that an opening at the inner circumferential side is formed at the proximal side with respect to an opening at the outer circumferential side. Thereby, the wall surface of the hole 33 at the distal side forms an inclined surface 33A which makes an acute angle with respect to the axis. The wall surface of the hole 33 at the proximal side forms an inclined surface 33B which makes an obtuse angle with respect to the axis.

A pair of the second diameter-enlarged portions 32 at the proximal side is also symmetrically provided across the hole 25A, and includes holes 35 which are bored toward the diameter-enlarged hole 25A from the outer periphery of the operation part body 21. Each hole 35 is bored obliquely with respect to the axis of the operation part body 21 so that an opening at the inner circumferential side is formed at the distal side with respect to an opening at the outer circumferential side. Thereby, the wall surface of the hole 35 at the distal side forms an inclined surface 35A which makes an obtuse angle with respect to the axis. The wall surface of the hole 35 at the proximal side forms an inclined surface 35B which makes an acute angle with respect to the axis.

In the operation tube 22, a mouthpiece 41 is integrally formed at a proximal end of the tube portion 26 which can be inserted into the hole 25A. The operation tube 22 is provided with a through hole 42 which leads to the tip of the tube portion 26 from the mouthpiece 41. A hard pipe 43 is press-fitted into and fixed to a distal end of the through hole 42. The pipe 43 advances into the hole 25 of the operation part body 21, and is connected to the tube 9.

As shown in FIG 7, two keys 44 are provided at the outer periphery of the tube portion 26 so as to protrude therefrom. The keys 44 are formed in accordance with the formation positions of the key grooves 27 at the operation part body 21. The relative rotation of the keys and the key grooves around the axis is prevented by inserting the operation tube 22 into the operation part body 21 so that the keys 44 engage the key grooves 27.

As shown in FIG. 6, a pair of locking portions 51 is formed at the tip of the operation tube 22 so as to pinch the pipe 43. The pair of locking portions 51 is portions which are formed so as to be elastically deformable by making a slit in the tip of the tube portion 26, and has protruding portions 52 provided on the radial outside at distal ends which become free ends. Each protruding portion 52 has an inclined surface 52A of which the tip is obliquely cut toward the center. The proximal side of the protruding portion 52 has become a flat surface 52B substantially along the radial direction. In a state where an external force does not act, the distance between external surfaces 52C of the pair of protruding portions is greater than the diameter of the hole 25A.

The locking portions 51 and the diameter-enlarged portions 31 and 32 are formed so that the needle tube 5 projects from the sheath 3, and is located at a working position where the needle tube can be inserted into a tissue, when the protruding portions 52 of the locking portions 51 are made to coincide with first diameter-enlarged portions 31. Furthermore, the locking portions 51 and the diameter-enlarged portions 31 and 32 are formed so that the needle tube 5 is located at a housed position where the needle tube 5 is completely pulled into the sheath 3, when the protruding portions 52 of the locking portions 51 are made to coincide with the second diameter-enlarged portions 32.

The operation when the treatment tool 1 constructed as described above is used will be described below.

First, a user inserts an endoscope (not shown) through a patient's mouth or the like, and moves the tip of the endoscope to a part to be treated while confirming with an imaging device of the endoscope.

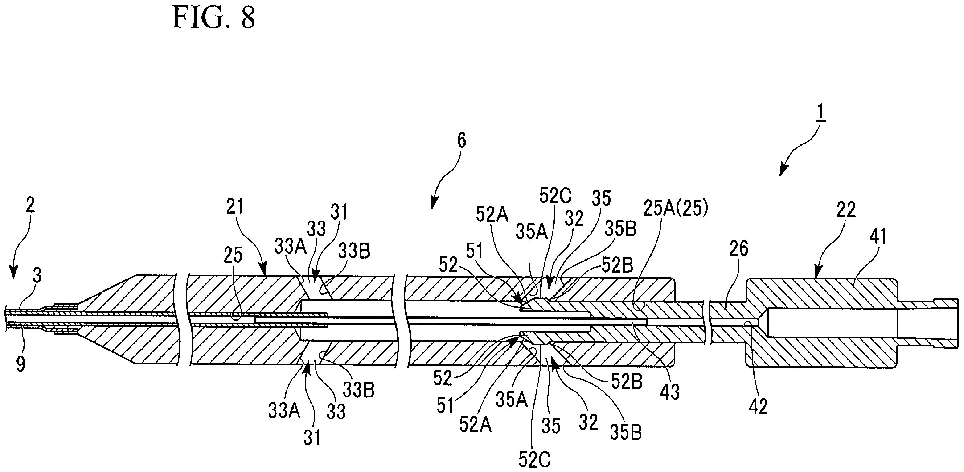

Next, the treatment tool 1 is inserted through a working channel of the endoscope, and the tip of the sheath 3 of the insertion portion 2 is endoscopically introduced into a body cavity. As shown in FIG 8, as an initial state of the operation part 6, the protruding portions 52 of the locking portions 51 are locked to the second diameter-enlarged portions 32, and the needle tube 5 is housed in the sheath 3. Since the flat surfaces 52B of the protruding portions 52 abut on the inclined surfaces 35B of the second diameter-enlarged portions 32 at this position, it is no longer possible to pull out the operation tube 22.

When the needle tube 5 is projected from the sheath 3, the user holds the mouthpiece 41 and pushes the operation tube 22 into the operation part body 21. By means of the inclined surfaces 35A of the second diameter-enlarged portions 32 at the distal side, the pair of locking portions 51 is pressed in a direction in which the locking portions are closed (radially inward of the tube portion 26), and the distance between the external surfaces 52C of the protruding portions 52 is decreased less than or equal to the diameter of the hole 25A. As a result, the pair of locking portions 51 can be advanced into the hole 25A. When the operation tube 22 is pushed in, the needle tube 5 coupled with the operation tube 22 via the pipe 43 or the tube 9 advances with respect to the sheath 3.

Since the diameter of the hole 25A is substantially constant to the first diameter-enlarged portions 31, the operation tube 22 advances smoothly, and the needle tube 5 begins to project from the sheath 3. When the protruding portions 52 of the pair of locking portions 51 reach the first diameter-enlarged portions 31, the pair of locking portions 51 return to their original positions and separate from each other, and as shown in FIG. 6, the locking portions 51 are received in the first diameter-enlarged portions 31. When the operation tube 22 is at this position, the needle tube 5 is projected from the sheath 3 at the distal side by a predetermined length.

Here, in a case where the operation tube 22 is intended to further advance, the inclined surfaces 52A at the tips of the protruding portions 52 abut on the inclined surfaces 33A of the first diameter-enlarged portions 31. Since the inclination direction of the inclined surfaces 33A is a direction in which the inclined surfaces 33A do not press the locking portions 51 in a diameter-reduced direction, but interfere with the locking portion, the operation tube 22 cannot be advanced any further.

On the other hand, when the operation tube 22 is intended to be pulled and retracted by a comparatively weak force, the surfaces 52B of the protruding portions 52 at the proximal side interfere with the inclined surfaces 33B of the first diameter-enlarged portions 31 by abutting thereto. That is, the locking portions 51 are locked to the first diameter-enlarged portions 31, and the position of the needle tube 5 is locked. Accordingly, if a user pushes in the operation part body 21 to advance the whole treatment tool 1, the needle tube 5 can be inserted into a tissue to be treated.

At this time, since the external diameter of the large diameter portion 8A of the coupling member 8 integrally fixed to the needle tube 5 is set to be equal to or slightly smaller than the internal diameter of the sheath 3, almost no gap exists between both the large diameter portion 8A and the sheath 3. Thereby, even if a force acts on the needle tube 5 at the time of procedure, such as insertion, the needle tube 5 does not wobble so as to separate from the axis of the sheath 3, and the coupling member 8 smoothly slides inside the sheath 3.

If the needle tube 5 has been inserted into a tissue, fluids, such as various liquids, such as a medicinal solution and a saline solution, various gases, such as air, or the like are delivered from a syringe (not shown) connected to the mouthpiece 41. A supplied fluid is injected into the tissue from the needle tube 5 through the tube 9. Thereby, various kinds of procedures, such as drug administration and tissue swelling, can be performed.

After the end of a treatment, a user retracts the treatment tool 1 to extract the needle tube 5 from the tissue.

When the needle tube 5 is housed into the sheath 3, a user pulls the operation tube 22 with a stronger force than an acting force at the time of insertion, and retracts the operation tube 22 with respect to the operation part body 21. The protruding portions 52 of the locking portions 51 are pressed by the inclined surfaces 33B of the first diameter-enlarged portions 31, and the pair of locking portions 51 is closed. This enables the operation tube 22 to retract. The engagement force between the protruding portions 52 and the inclined surfaces 33B regulates unintended movement of the operation tube 22 when the needle tube 5 is inserted into a tissue, and the shape of the operation tube 22 is set so as to deform the pair of locking portions 51 when the mouthpiece 41 is manually pulled.

When the protruding portions 52 reach the second diameter-enlarged portions 32 in the process in which the operation tube 22 is retracted, the pair of locking portions 51 returns to its original position, and is caught in the operation part body 21. When the locking portions 51 are stopped at this position, the needle tube 5 is completely housed in the sheath 3. In addition, the inclined surfaces 35B of the second diameter-enlarged portions 32 at the proximal side interfere with the locking portions 51 in a direction in which the operation tube 22 retracts, and prevent slip-off of the locking portions. Accordingly, the operation tube 22 is not caused to slip off by normal operation.

According to the treatment tool 1 of the present embodiment, in the coupling member 8, the needle tube 5 and the coupling member 8 are firmly and integrally fixed by caulking the fixing portion 8B with a smaller external diameter than the large diameter portion 8A. In a fixed state, the maximum external diameter of the caulked fixing portion 8B is set to be less than or equal to the external diameter of the large diameter portion 8A.

Accordingly, since the external diameter of the coupling member 8 is always greatest at the large diameter portion 8A which is not deformed even after caulking, almost no gap exists between the large diameter portion 8A and the sheath 3 whose cross-sections in the radial direction are substantially true circles. Therefore, the coupling member 8 and the needle tube 5 slide smoothly without clattering within the sheath 3, and do not wobble so that the needle tube 5 separates from the axis of the sheath 3. As a result, the behavior of the needle tube 5 serving as the treatment part 4 can be consistently stabilized during a procedure, and the procedure can be easily and reliably performed.

Additionally, in the coupling member 8, the large diameter portion 8A which specifies a maximum external diameter, and the fixing portion 8B used for caulking fixing are formed in separate regions. Thus, the thickness of the fixing portion 8B can be set to a wall thickness which is always optimal for caulking irrespective of the external diameter of the coupling member 8.

Additionally, the diameter of the opening 3A of the tip of the sheath 3 is set such that the coupling member 8 cannot pass therethrough. Thus, even if the operation part body 21 and the operation tube 22 are disengaged, unintended projection of the needle tube 5 can be prevented as the abutting portion 7 and the coupling member 8 abut each other.

Moreover, since the large diameter portion 8A is provided at the distal side of the fixing portion 8B, deformation of the fixing portion 8B caused by caulking is not transmitted to the distal side of the coupling member 8. Accordingly, in order to suppress clattering of the needle tube 5, it is not necessary to elongate the axial dimension of the fixing portion 8B, and the dimension, in the axis direction, of the region of the coupling member 8 closer to the distal side than the connecting portion 8C can be made shorter than before, for example, can be made shorter to about 2 millimeters (mm). As a result, the hard length (the length of a region of which the flexibility is relatively lower than the sheath 3 or the tube 9) of the treatment tool 1 at the distal side can be made short to improve the insertion performance of the treatment tool 1.

In addition, since two diameter-enlarged portions 31 and 32 are provided in the extending/retracting direction of the needle tube 5, and the diameter-enlarged portions 31 and 32 are formed with a tapered face, it is possible to deform the protruding portions 52 of the operation tube 22 simply by extending or retracting the operation tube 22. That is, operation becomes easy with a simple construction. Since the diameter-enlarged portions 31 and 32 are formed so as to correspond to a working position to which the needle tube 5 projects, and at the housed position where the needle tube 5 is completely housed in the sheath 3, projecting/receding of the needle tube 5 can be controlled simply by moving the operation tube 22 to its locked position.

In an injection needle of a type in which a needle tube is fixed to a working position by pushing or screwing a mouthpiece into an operation part body as in a conventional technique, a large amount of force required for pushing-in or screwing-in ultimately becomes necessary. In contrast, in this treatment tool 1, it is not ultimately necessary to apply a large amount of force, and thus, the operation is easy. Additionally, when the needle tube 5 is fixed to a working position, there is no longer a shortage in the required amount of force.

Subsequently, a treatment tool of a second embodiment of the invention will be described with reference to FIG 8. A difference between the treatment tool 61 of the present embodiment and the endoscopic treatment tool 1 of the above-described first embodiment is the shape of the coupling member. In addition, constituent elements common to those of the above-described treatment tool 1 will be designated by the same reference numerals, and the description thereof is omitted.

FIG 8 is an enlarged sectional view in the vicinity of the tip of the treatment tool 61. A coupling member 62 of the treatment tool 61 is provided with two large diameter portions 63 of a first large diameter portion 63A at the distal side and a second large diameter portion 63B adjacent to a connecting portion 64 connected to the tube 9. A fixing portion 65 which is caulked in order to integrally fix the needle tube 5 and the coupling member 62 is provided between the first large diameter portion 63A and the second large diameter portion 63B.

According to the treatment tool 61 of the present embodiment, the coupling member 62 is provided with two large diameter portions of the first large diameter portion 63A and the second large diameter portion 63B. Thus, wobbling of the needle tube 5 in the above-described procedure can be more suitably prevented.

Additionally, since the fixing portion 65 is located so as to be pinched by the large diameter portions 63A and 63B, the deformation caused by the caulking of the fixing portion 65 does not extend in any axis direction of the coupling member 62, and is limited to the fixing portion 65. Accordingly, it is not necessary to consider the deformation and set the fixing portion to be long. Therefore, it is possible to shorten the coupling member 62 to make the hard length of the tip of the treatment tool 61 shorter.

Moreover, although the coupling member 62 of the present embodiment is provided with two large diameter portions, the two large diameter portions are easily formed simply by performing cutting or the like of an outer circumferential surface so that the fixing portion is formed in a tubular member which has the same external diameter before working as the large diameter portions. Accordingly, it is possible to manufacture the coupling member 62 through substantially the same process as that of the above-described coupling member 8, and the manufacturing does not become complicated.

Although the embodiments of the invention have been described hitherto, the technical scope of the invention is not limited to the above embodiments, but various modifications may be made without departing from the scope of the invention.

An example in which the abutting portion is provided at the tip of the sheath has been described in the above embodiments. Instead of this, however, the abutting portion may be formed at a position separated from the tip by a predetermined length, for example, by a method of press-fitting a ring-shaped member into the sheath. In this case, the large diameter portion may not necessarily be provided at the tip of the coupling member.

Additionally, the abutting portion is not necessarily formed over the whole circumferential direction as long as the treatment part can pass therethrough and the coupling member cannot pass therethrough. For example, the abutting portion may be formed so as to protrude partially from the inner wall of the sheath so that the internal diameter thereof is just partially reduced.

Moreover, an example in which the treatment part and the tube which is a connecting member are integrally coupled together via the coupling member has been described in the above embodiments. Instead of this, however, the treatment part and the connecting member may be coupled together by other means, such as press-fitting, bonding, and welding, and a coupling member may be attached to either the treatment part or the connecting member, or both the treatment part and the connecting member as a stopper. Even in this way, it is possible to suppress wobbling of the treatment part or connecting member suitably.

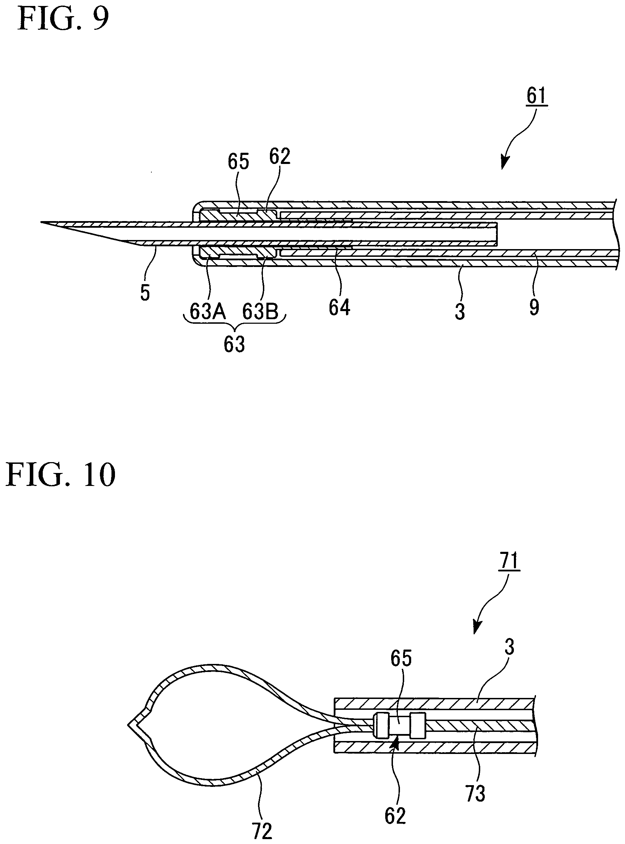

Additionally, an example in which a coupling member functions as a stopper abutting on the abutting portion has been described in the above-described embodiments. However, a configuration in which the abutting portion is not provided in the sheath 3, and the coupling member does not function as a stopper may be adopted like the treatment tool 71 of the modification shown in FIG 10.

In the treatment tool 71, a snare wire 72 is provided as a treatment part, and an operation wire 73 serving as a connecting member connects the snare wire 72 and an operation part (not shown). Ends of a snare wire 72 and an operation wire 73 are respectively inserted into both ends of a tubular coupling member 62, and, as the fixing portion 65 is fixed by caulking and brazing, the snare wire 72 and the operation wire 73 are integrally connected. Even in this configuration, wobbling of the snare wire 72 and the operation wire 73 can be suitably suppressed. Additionally, in the case of the treatment tool which takes such a configuration, the coupling member can be projected further forward than the tip of the sheath. Thus, for example, it is also possible to use the coupling member as a marker for roughly locating the treatment part under X-ray illumination.

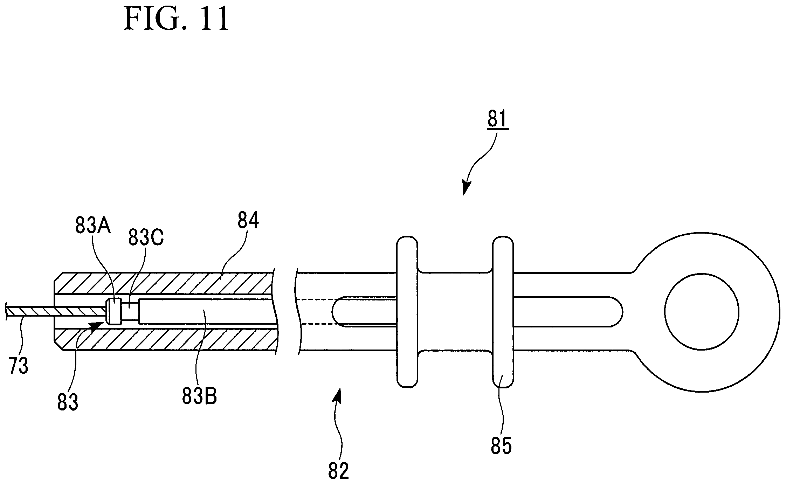

Moreover, as for the coupling member in the invention, a coupling member may be attached to the end of the operation wire 73, serving as a connecting member, at the side of the operation part 82, like the treatment tool 81 of the modification shown in FIG. 11.

In the treatment tool 81, the dimension of a coupling member 83 in the axis direction is set to be long, and the coupling member 83 functions as a buckling preventing pipe of the operation wire 73. The length, in the axis direction, of a second large diameter portion 83B at the proximal side is longer than a first large diameter portion 83A at the distal side. This makes it possible to suitably suppress wobbling of the operation wire 73 in the operation part body 84 of the operation part 82.

In addition, in this operation part 82, the operation wire 73 is connected to a slider 85 which is attached so as to be slidable in the axis direction of the operation part body 84, and extending//retracting operation of the operation wire 73 via the slider 85 is possible. However, an aspect in which the operation wire 73 and the slider 85 are connected together is not particularly limited. For example, the coupling member 83 is fixed to the operation wire 73 by fixing, such as caulking or brazing of a fixing portion 83C. However, the operation wire 73 may be directly connected to the slider 85 without being fixed to the coupling member 83, or the operation wire 73 and the slider 85 may be integrally connected via the coupling member 83. Moreover, both the operation wire 73 and the coupling member 83 may be connected to the slider 85.

In addition, although an example of the treatment tool which has the needle tube and the snare wire as the treatment part has been described in the above-described embodiments, the treatment tool to which the invention can be applied is not limited to this. Accordingly, the structure of the invention can be applied to various treatment tools including not only the needle tube and the above-described snare wire, but also various kinds of structures for performing arbitrary procedures as the treatment part.

### Industrial Applicability

According to the treatment tool of the invention, it is possible to provide a construction in which wobbling does not easily occur in a direction in which the treatment part or the connecting member separates from the axis.

### Reference Signs List

- 1, 61, 71, 81:: TREATMENT TOOL
- 3:: SHEATH
- 4:: TREATMENT PART
- 5:: NEEDLE TUBE
- 6, 82:: OPERATION PART
- 7:: ABUTTING PORTION
- 8, 62, 83:: COUPLING MEMBER (SLIDING MEMBER)
- 8A, 63:: LARGE DIAMETER PORTION
- 8B, 65, 8C:: FIXING PORTION
- 9:: TUBE (CONNECTING MEMBER)
- 63A, 83A:: FIRST LARGE DIAMETER PORTION
- 63B, 83B:: SECOND LARGE DIAMETER PORTION
- 72:: SNARE WIRE (TREATMENT PART)
- 73:: OPERATION WIRE (CONNECTING MEMBER)

## Claims

1. A treatment tool (1) for endoscopic insertion into a body cavity, comprising:
a treatment part (4) configured to perform a treatment within the body cavity;
a connecting member (9) connected to a proximal end of the treatment part (4);
a sheath (3) through which the treatment part (4) and the connecting member (9) are inserted so as to be able to extend or retract in an axis direction;
an operation part (6) which is connected to the connecting member (9); and
a coupling member (8) fixed to the treatment part (4) by caulking,
**characterized in that** the coupling member (8) has a large diameter portion (8A) having an external diameter (D2) equal to the internal diameter of the sheath (3) such that no gap exists between the large diameter portion (8A) and the sheath (3) thereby allowing the coupling member (8) and the treatment part (4) slide smoothly without clattering within the sheath (3), the coupling member (8) further has a fixing portion (8B) that is caulked for fixing the coupling member (8) to the treatment part (4) and has a smaller external diameter before caulking than the external diameter (D2) of the large diameter portion (8A), has a connecting portion (8C) having a smaller external diameter than that of the fixing portion (8B), and is formed in a tubular shape, and wherein the radial maximum dimension (D1) of the fixing portion (8B) after caulking is less than or equal to the external diameter (D2) of the large diameter portion (8A).

2. The treatment tool (1) according to Claim 1,
wherein the treatment part (4) and the connecting member (9) are integrally connected by the coupling member (8).

3. The treatment tool (1) according to Claim 1 or 2,
wherein the sheath (3) has an abutting portion (7) which is formed so that the treatment part (4) can pass therethrough and the coupling member (8) cannot pass therethrough.

4. The treatment tool (1) according to Claim 3,
wherein the abutting portion (7) is formed by reducing the internal diameter of the tip of the sheath (3).

5. The treatment tool (1) according to Claim 1 or 2,
wherein the large diameter portion has a first large diameter portion (63A) at the distal side, and a second large diameter portion (63B) located closer to the proximal side than the first large diameter portion (63A), and the fixing portion (65) is provided between the first large diameter portion (63A) and the second large diameter portion (63B).

6. The treatment tool (1) according to Claim 3,
wherein the large diameter portion has a first large diameter portion (63A) at the distal side, and a second large diameter portion (63B) located closer to the proximal side than the first large diameter portion (63A), and the fixing portion (65) is provided between the first large diameter portion (63A) and the second large diameter portion (63B).

7. The treatment tool (1) according to Claim 4,
wherein the large diameter portion has a first large diameter portion (63A) at the distal side, and a second large diameter portion (63B) located closer to the proximal side than the first large diameter portion (63A), and the fixing portion (65) is provided between the first large diameter portion (63A) and the second large diameter portion (63B).

8. The treatment tool (1) according to Claim 1, wherein the treatment part (4) is a hollow needle tube (5), and the connecting member (9) is formed in the shape of a tube capable of supplying a fluid to the needle tube (5).

## Patentansprüche

1. Behandlungswerkzeug (1) zum endoskopischen Einführen in einen Körperhohlraum, umfassend:
einen Behandlungsteil (4), der dazu eingerichtet ist, eine Behandlung innerhalb des Körperhohlraums vorzunehmen;
ein Verbindungselement (9), das mit einem proximalen Ende des Behandlungsteils (4) verbunden ist;
eine Hülle (3), durch die der Behandlungsteil (4) und das Verbindungselement (9) derart eingeführt werden können, dass sie in der Lage sind, in einer Achsrichtung hervorzustehen oder zurückversetzt zu werden;
ein Betätigungsteil (6), das mit dem Verbindungselement (9) verbunden ist; und
ein Kupplungselement (8), das an dem Behandlungsteil (4) durch Verstemmen befestigt ist,
**dadurch gekennzeichnet, dass** das Kupplungselement (8) einen Abschnitt (8A) mit einem großen Durchmesser aufweist, der einen Außendurchmesser (D2) aufweist, der gleich dem Innendurchmesser der Hülle (3) ist, so dass keine Lücke zwischen dem Abschnitt (8A) mit dem großen Durchmesser und der Hülle (3) entsteht, wodurch es dem Kupplungselement (8) und dem Behandlungsteil (4) ermöglicht wird, gleichmäßig ohne Verkippen innerhalb der Hülle (3) zu gleiten,
wobei das Kupplungselement (8) ferner einen Befestigungsabschnitt (8B) aufweist, der verstemmt wird, um das Kupplungselement (8) an dem Behandlungsteil (4) zu befestigen und der einen kleineren Außendurchmesser vor dem Verstemmen aufweist, als der Außendurchmesser (D2) des Abschnitts (8A) mit dem großen Durchmesser, der einen Verbindungsabschnitt (8C) aufweist, der einen geringeren Außendurchmesser aufweist als derjenige des Befestigungsabschnitts (8B) und der mit einer röhrenförmigen Form ausgebildet ist, und wobei die radiale maximale Abmessung (D1) des Befestigungsabschnitts (8B) nach dem Verstemmen geringer als oder gleich zu dem Außendurchmesser (D2) des Abschnitts (8A) mit dem großen Durchmesser ist.

2. Behandlungswerkzeug (1) nach Anspruch 1,
wobei der Behandlungsteil (4) und das Verbindungselement (9) durch das Kupplungselement (8) integral miteinander verbunden sind.

3. Behandlungswerkzeug (1) nach Anspruch 1 oder 2,
wobei die Hülle (3) einen Anschlagabschnitt (7) aufweist, der derart geformt ist, dass der Behandlungsteil (4) dort hindurch passt und das Kupplungselement (8) dort nicht hindurch passt.

4. Behandlungswerkzeug (1) nach Anspruch 3,
wobei der Anschlagabschnitt (7) durch Verringern des Innendurchmessers einer Spitze der Hülle (3) gebildet wird.

5. Behandlungswerkzeug (1) nach Anspruch 1 oder 2,
wobei der Abschnitt mit dem großen Durchmesser einen ersten Abschnitt (63A) mit einem großen Durchmesser an einer distalen Seite aufweist und einen zweiten Abschnitt (63B) mit einem großen Durchmesser, der näher an der proximalen Seite als der erste Abschnitt (63A) mit einem großen Durchmesser angeordnet ist, und wobei der Befestigungsabschnitt (65) zwischen dem ersten Abschnitt (63A) mit dem großen Durchmesser und dem zweiten Abschnitt (63B) mit dem großen Durchmesser angeordnet ist.

6. Behandlungswerkzeug (1) nach Anspruch 3,
wobei der Abschnitt mit dem großen Durchmesser einen ersten Abschnitt (63A) mit einem großen Durchmesser an der distalen Seite und einen zweiten Abschnitt (63B) mit einem großen Durchmesser aufweist, der näher an der proximalen Seite angeordnet ist als der erste Abschnitt (63A) mit dem großen Durchmesser, und wobei der Befestigungsabschnitt (65) zwischen dem ersten Abschnitt (63A) mit dem großen Durchmesser und dem zweiten Abschnitt (63B) mit dem großen Durchmesser angeordnet ist.

7. Behandlungswerkzeug (1) nach Anspruch 4,
wobei der Abschnitt mit dem großen Durchmesser einen ersten Abschnitt (63A) mit einem großen Durchmesser an der distalen Seite und einen zweiten Abschnitt (63B) mit einem großen Durchmesser aufweist, der näher an der proximalen Seite angeordnet ist als der erste Abschnitt (63A) mit dem großen Durchmesser, und wobei der Befestigungsabschnitt (65) zwischen dem ersten Abschnitt (63A) mit dem großen Durchmesser und dem zweiten Abschnitt (63B) mit dem großen Durchmesser angeordnet ist.

8. Behandlungswerkzeug nach Anspruch 1, wobei der Behandlungsteil (4) ein hohles Nadelrohr (5) ist und das Verbindungselement (9) in Form eines Rohrs ausgebildet ist, das dazu ausgebildet ist, ein Fluid zu dem Nadelrohr (5) zu fördern.

## Revendications

1. Outil (1) de traitement pour insertion endoscopique à l'intérieur d'une cavité corporelle, comprenant :
une partie (4) de traitement configurée pour exécuter un traitement à l'intérieur de la cavité corporelle ;
un élément (9) de connexion connecté à une extrémité proximale de la partie (4) de traitement ;
une gaine (3) à travers laquelle la partie (4) de traitement et l'élément (9) de connexion sont insérés de manière à être aptes à s'étendre ou se rétracter dans un sens axial ;
une partie (6) opérationnelle qui est connectée à l'élément (9) de connexion ; et
un élément (8) de couplage fixé à la partie (4) de traitement par calfeutrage,
**caractérisé en ce que** l'élément (8) de couplage a une partie (8A) de grand diamètre ayant un diamètre externe (D2) égal au diamètre interne de la gaine (3) de telle sorte qu'aucun espace n'existe entre la partie (8A) de grand diamètre et la gaine (3), permettant ainsi que l'élément (8) de couplage et la partie (4) de traitement coulissent de façon régulière sans cliquetis à l'intérieur de la gaine (3), l'élément (8) de couplage a en outre une partie (8B) de fixation qui est calfeutrée pour fixer l'élément (8) de couplage à la partie (4) de traitement et a un diamètre externe avant calfeutrage plus petit que le diamètre externe (D2) de la partie (8A) de grand diamètre, a une partie (8C) de connexion ayant un diamètre externe plus petit que celui de la partie (8B) de fixation, et est formée à une forme tubulaire, et dans lequel la dimension radiale maximum (D1) de la partie (8B) de fixation après calfeutrage est inférieure ou égale au diamètre externe (D2) de la partie (8A) de grand diamètre.

2. Outil (1) de traitement selon la revendication 1,
dans lequel la partie (4) de traitement et l'élément (9) de connexion sont connectés de façon intégrale par l'élément (8) de couplage.

3. Outil (1) de traitement selon la revendication 1 ou 2,
dans lequel la gaine (3) a une partie (7) de butée qui est formée de telle manière que la partie (4) de traitement peut passer à travers celle-ci et que l'élément (8) de couplage ne peut pas passer à travers celle-ci.

4. Outil (1) de traitement selon la revendication 3,
dans lequel la partie (7) de butée est formée en réduisant le diamètre interne de la pointe de la gaine (3).

5. Outil (1) de traitement selon la revendication 1 ou 2,
dans lequel la partie de grand diamètre a une première partie (63A) de grand diamètre au niveau du côté distal, et une deuxième partie (63B) de grand diamètre située plus près du côté proximal que la première partie (63A) de grand diamètre, et la partie (65) de fixation est prévue entre la première partie (63A) de grand diamètre et la deuxième partie (63B) de grand diamètre.

6. Outil (1) de traitement selon la revendication 3,
dans lequel la partie de grand diamètre a une première partie (63A) de grand diamètre au niveau du côté distal, et une deuxième partie (63B) de grand diamètre située plus près du côté proximal que la première partie (63A) de grand diamètre, et la partie (65) de fixation est prévue entre la première partie (63A) de grand diamètre et la deuxième partie (63B) de grand diamètre.

7. Outil (1) de traitement selon la revendication 4,
dans lequel la partie de grand diamètre a une première partie (63A) de grand diamètre au niveau du côté distal, et une deuxième partie (63B) de grand diamètre située plus près du côté proximal que la première partie (63A) de grand diamètre, et la partie (65) de fixation est prévue entre la première partie (63A) de grand diamètre et la deuxième partie (63B) de grand diamètre.

8. Outil (1) de traitement selon la revendication 1, dans lequel la partie (4) de traitement est un tube à aiguille (5) creux, et l'élément (9) de connexion est formé à la forme d'un tube apte à amener un fluide jusqu'au tube à aiguille (5).
